# EUROPEAN PATENT APPLICATION

(11) **EP 0 729 030 A1**
(43) Date of publication of application: **28.08.1996**
(21) Application number: 96301173.9
(22) Date of filing: 21.02.1996
(51) Int. Cl.: G01N 33/58, G01N 33/543, G01N 33/537

(54) **Chemiluminescence method**

(30) Priority: 23.02.1995 JP 62067/95
(71) Applicant: KYOTO DAIICHI KAGAKU CO., LTD., Kyoto-shi Kyoto-fu (JP)
(72) Inventor: Yonehara, Satoshi, c/o Kyoto Daiichi Kagaku Co Ltd, Kyoto-shi, Kyoto-fu, 601 (JP); Takama, Toshio, c/o Kyoto Daiichi Kagaku Co Ltd, Kyoto-shi, Kyoto-fu, 601 (JP)
(74) Representative: Gaunt, Robert John

(57) **Abstract**

An immunoassay method using a chemiluminescent acridinium ester label for quantitatively determining the amount of a substance, typically an antigen or an antibody, in a sample. The method comprises the following steps in sequence:-
a) optionally, diluting the sample with a diluent liquid to a desired concentration,
b) bonding a reagent containing an acridinium ester-labeled antibody (or antigen) capable of specifically recognizing the substance to be measured in the sample and a physiologically-active substance that has been treated so as to be able to be fixed on a solid phase, to the substance to be measured in the sample by antigen-antibody reaction to thereby form an immunocomplex,
c) fixing the immunocomplex on a solid phase,
d) separating the antibody (or antigen) that has not participated in the reaction from the reacted acridinium ester-labeled antibody (or antigen), using a washing liquid, while converting the acridinium ester of a non-luminescent type into that of a luminescent type, and
e) adding an alkaline solution to the reaction system to thereby make the system emit light, followed by measuring the amount of the light thus emitted. The method is characterized in that, of the diluent liquid to be used in the step a), the reagent to be used in the step b) and the washing liquid to be used in the step d), at least the washing liquid has a pH of from 5 to 6.

## Description

The present invention relates to a method of immunoassay by heterogeneous chemiluminescence using a chemiluminescent substance, and more precisely, it relates to a method of immunoassay by acridinium ester -labeled chemiluminescence in which the liquid treating agents to be applied to the method are optimized thereby to simplify the operations for the method and to improve the accuracy in the data to be measured by the method.

Immunoassay includes a homogeneous process and a heterogeneous process. Of these, the latter heterogeneous process is employed for measuring minor components such as tumor markers, hormones, etc., by which it is possible to measure such components at high sensitivity. According to the heterogeneous process for measuring the amount of an antigen, in general, a physiologically - active substance (for example, antibody, enzyme, inhibitor, etc.) that can specifically recognize the antigen to be measured and that has been previously fixed on a solid phase, the antigen to be measured, and an antibody that can specifically recognize the antigen and that has been detectably labeled are mixed in a container and kept under predetermined conditions, to thereby produce a complex of the physiologically - active substance, the antigen and the labeled antibody. Since the complex is bonded to a solid phase, the reaction system is then washed so that the non - reacted, detectably - labeled antibody is separated from the reaction system. After this, the detectably - labeled amount of the thus - separated antibody is measured, from which the amount of the antigen to be measured is determined.

The detectable labeling substance includes enzymes, fluorescent substances, luminescent substances, radioactive isotopes, etc. Of these, enzymes, fluorescent substances and luminescent substances are popularly used as being advantageous in that they are safely handled. However, the method of using enzymes is defective in that the reaction of enzymes and the substrates needs a fairly long period of time and therefore it is impossible to reduce the time necessary for the measurement. The method of using fluorescent substances is also defective in that the sensitivity is limited and therefore high - sensitivity measurement is impossible. As opposed to these, the method of using luminescent substances is advantageous in that the measurement can be finished within a short period of time and that high - sensitivity measurement is possible, and therefore this is more popularly used.

As luminescent substances, chemiluminescent substances are generally employed since they can easily be handled. As chemiluminescent substances, generally known are acridinium esters, luminol, isoluminol, oxalates, etc. These luminescent substances generally emit light when they are in alkaline solutions containing hydrogen peroxide.

The method of using acridinium esters as chemiluminescent substances is effective. The use of acridinium esters is described in Clinical Chemistry, 29/8, 1474 - 1479 (1983), and the sensitivity thereof is comparable to or higher than that of a labeling substance of a radioactive isotope, I125.

in order to make acridinium esters emit light, in general, the following process is necessary. First, an acidifying buffer solution is added to a solution containing an acridinium ester to make the latter solution acid, then the resulting acid solution is kept as it is for a predetermined period of time, and thereafter an alkaline solution containing hydrogen peroxide is added thereto to induce the emission of light. It is known that acridinium esters of a non - luminescent type are converted into those of a luminescent type under acidic conditions and that the amount of the non - luminescent acridinium ester to be converted into luminescent one increases greater at a lower pH value. For these reasons, sufficient acidic conditions are necessary in order to conduct high - sensitivity measurement by the use of acridinium esters. On the other hand, it is generally known that antigen-antibody bonds become easily dissociated under acidic conditions.

Since acridinium esters have the properties as mentioned above, the immunoassay using acridinium esters is generally carried out according to the process mentioned below.

First a physiologically - active substance that can specifically recognize an antigen to be measured and that has been previously fixed on a solid phase, an antigen to be measured, and an antibody that can specifically recognize the antigen to be measured and that has been labeled with an acridinium ester are mixed in a container and kept under predetermined conditions, to thereby produce a complex of the physiologically -active substance, the antigen and the labeled antibody. Since the complex is bonded to a solid phase, the reaction system is then washed so that the non-reacted, acridinium ester - labeled antibody is separated from the reaction system. Next, an acidifying buffer solution having pH of 4 or lower is added to the thus - separated antibody and kept for a predetermined period of time whereby the acridinium ester of a non - luminescent type in the system is converted into that of a luminescent type. Next, an alkaline solution containing hydrogen peroxide is added to the resulting acid system to make it emit light. The amount of the light emitted is measured, from which the amount of the antigen to be measured is determined.

However, the above - mentioned prior art process has some problems in the following points.
(1) Since the antigen-antibody bond in the reaction system is dissociated due to the addition of the acidifying buffer solution to the system, some means of preventing the dissociation of the antigen-antibody bond shall be applied to the system.
(2) Since the mere addition of the acidifying buffer solution to the reaction system could not attain the sufficient penetration of the solution into the fixed reaction system especially when a fibrous aggregate is used as the carrier in the solid phase, it takes a long period of time to convert the acridinium ester of a non - luminescent type into that of a luminescent type.
(3) Since the alkaline solution added is rapidly neutralized by the already - existing, acidifying buffer solution having a low pH value, sufficient emission of light could not often been attained.

The object of the present invention is to provide washing liquids for chemiluminescence capable of solving the problems which the prior art process has.

We, the present inventors have assiduously studied so as to solve the above - mentioned problems in the prior art and, as a result, have attained the present invention of an immunological chemiluminescence method using an acridinium ester as the labeling substance for quantitatively determining the antigen (or substance to be measured) to be contained in a sample, which comprises the following steps in that order:
(a) a step of diluting the sample with a diluent liquid to a desired concentration,
(b) a step of bonding a reagent containing an acridinium ester-labeled antibody (or antigen) capable of specifically recognizing the substance to be measured to be in the sample and a physiologically - active substance that has been treated so as to be able to be fixed on a solid phase, to the substance to be measured to be in the sample by antigen - antibody reaction to thereby form an immunocomplex,
(c) a step of fixing the immunocomplex on a solid phase,
(d) a step of separating the antibody (or antigen) that has not participated in the reaction from the reacted, acridinium ester-labeled antibody (or antigen), using a washing liquid, while converting the acridinium ester of a non - luminescent type into that of a luminescent type, and
(e) a step of adding an alkaline solution to the reaction system to thereby make the system emit light, followed by measuring the amount of the light thus emitted, the method being characterized in that, of the diluent liquid to be used in the step (a), the reagent to be used in the step (b) and the washing liquid to be used in the step (d), at least the washing liquid has pH of from 5 to 6.

If the concentration of the substance to be measured in the sample is extremely low, the dilution is unnecessary and therefore the step (a) may be omitted.

The method of the present invention is, being different from the prior art process, characterized in that one and the same liquid that acts as both the washing liquid and the acidifying buffer solution to be employed in the prior art process is used. However, if a single conventional washing liquid that may act also as an acidifying buffer solution is used in the method of the invention, the immunocomplex formed is dissociated under acidifying conditions such as those mentioned above with the result that the amount of light to be emitted is lowered and the data measured shall vary.

In consideration of the problem, we, the present inventors have further studied so as to specifically define the pH range suitable for the single washing liquid employable in the present invention, with which it is possible to sufficiently convert the acridinium ester of a non - luminescent type into that of a luminescent type without substantially causing the dissociation of the antigen - antibody bond in the reaction system and which can contain a buffer capable of exhibiting its buffering capacity within the thus -defined pH range. Concretely, we have studied so as to specifically define the pH range suitable for the single washing liquid employable in the present invention, with which the acridinium ester of a non -luminescent type can be converted into that of a luminescent type while the antigen - antibody bond in the reaction system is not dissociated. After all, we, the present inventors have found that a washing liquid having pH of from 5.0 to 6.0 meets the intended object of the present invention.

Any and every buffer that can exhibit its buffering capacity within the defined pH range can be used in the present invention. For example, the buffer includes water - soluble organic acids and inorganic acids, such as citric acid, maleic acid, acetic acid, phosphoric acid, boric acid, carbonic acid, etc. their hydroacids and water - soluble salts, etc. These can be used singly or as combined. The concentration of the buffer to be used is not specifically defined, provided that the buffer maintains its buffering capacity.

The washing step in the method of the present invention can be conducted entirely in the same manner as in conventional washing steps, provided that it is conducted within the defined pH range within which the antigen - antibody bond in the system is not dissociated. The residue that shall remain after the washing is not specifically defined. However, since the acridinium ester exists on the surface of the solid phase while it is fixed thereon, it is desirable that a thin filmy residue remains on the surface of the washed solid phase.

In the method of the present invention, it is desirable that the chemiluminescence initiator of an acidifying substance (for example, hydrogen peroxide) is added to the system along with the washing liquid and /or the alkaline solution. The amount of the initiator may be such that it is enough to make the acridinium ester emit light. It is desirable that the amount is not less than 10 times the amount of the acridinium ester as remained after the washing step. Any known acridinium esters can be used in the method of the present invention. As one example of the compounds, mentioned is Acridinium I (chemical name: 4-(2 -succinimidyloxycarbonyl) phenyl - 10 - methylacridinium -9-carboxylate fluorosulfonate; product of Dojin Chemical Co.).

The carrier for the solid phase to be used in the present invention includes beads, test tubes, microplates, microparticles, glass fiber aggregates (e.g., glass fiber filter paper), cellulose fiber aggregates, etc. Of these, especially preferred are glass fiber aggregates. This is because glass is inactive on the substances to be measured and the reagents used, the surface area of fibrous aggregates of glass is larger than that of solid glass when the volume of the former is the same as that of the latter and therefore the ability of the former to retain therewith the substances to be measured and the reagents used is higher than that of the latter, and, in addition, glass fiber aggregates have good light permeability therethrough in the detection of emitted light and fluorescent light using them.

Since the pH value of the system to be washed according to the present invention is defined to be such that the antigen - antibody bond in the system is not dissociated at the defined pH value, it is possible to prevent the variation in the values measured that shall be caused by the dropping of the acridinium ester-labeled antibody from the solid phase.

Since the acridinium ester of a non-luminescent type is converted into that of a luminescent type while the system is washed according to the present invention, even the acridinium ester of a non - luminescent type that exists in the part through which the alkaline solution added only once thereto could not sufficiently penetrate can be converted efficiently into that of a luminescent type.

In addition, since the two steps of washing and acidifying are combined into one step according to method of the present invention, the method is simplified and the time necessary for conducting the method can be shortened.

Moreover, since the washing liquid used is forcedly removed by suction under reduced pressure according to the present invention, the amount of the washing liquid still remained in the system even at the step of adding the alkaline solution to the system. Therefore, the titer value of the alkaline solution added as a luminescence initiator is not almost lowered. Accordingly, the light - emitting reaction of the acridinium ester in the system is more efficiently promoted.

Embodiments of the present invention are described hereinunder by means of the following examples, which, however, do not whatsoever restrict the technical scope of the present invention.

### Example 1:

Alpha - fetoprotein (hereinafter referred to as AFP) was measured in accordance with the following method where the glass fiber aggregate as described in Japanese Patent Laid - Open No. 4 - 203968 was used.1) Preparation of avidin-bonded glass fiber filter paper:

A glass fiber filter paper (F145 - 03 having a thickness of 1.3 mm ; produced by Wattman Co.) was dipped in a 2 % acetone solution of 3-aminopropyltriethoxysilane for 30 minutes at room temperature, whereby the glass was reacted with 3-aminopropyltriethoxysilane. Thus, an aminated glass fiber filter paper was obtained. The aminated glass fiber filter paper was dipped in a 0.1 mol/ liter sodium phosphate buffer (hereinafter referred to as NaPB) (pH 8.0) containing 0.0125 mg/ml of NHS - LC - biotin (produced by Dojin Chemical Co.), for 30 minutes at room temperature and reacted. After washed with distilled water, the biotin - introduced glass fiber filter paper was dipped in 0.1 mol/liter NaPB (pH 7.0) containing 0.1 mg/ml of avidin, for 30 minutes at room temperature. After washed with distilled water, the avidin- introduced glass fiber filter paper was dipped in a solution of Blockace (produced by Snow Brand Milk Products Co.) for 30 minutes at room temperature. Thus, an avidin - bonded glass filter paper was obtained.

### 2) Preparation of Acridinium I-labeled anti-AFP antibody:

An anti-AFP antibody (produced by KYOTO DAI - ICHI KAGAKU Co.) was dissolved in 0.1 mol/liter NaPB (pH 8.0) to prepare its solution having a concentration of 1 mg/liter. Acridinium I was dissolved in dimethylsulfoxide to prepare its solution having a concentration of 0.5 mol/liter. DL-lysine was dissolved in 0.1 mol/liter NaPB (pH 8.0) to prepare its solution having a concentration of 50 g/liter. 0.1 ml of the solution of Acridinium I was added to 0.9 ml of the solution of anti-AFP antibody and reacted for 30 minutes at room temperature. 0.1 ml of the solution of DL - lysine was added thereto and reacted for 15 minutes at room temperature. Using Ultrafree C3.C1 (produced by Millipore Co.), the reaction mixture was washed with 0.1 mol/liter NaPB (pH 8.0) to make it have a concentration of 0.001 mg/ml. Thus, an Acridinium I-labeled anti-AFP antibody was obtained.

### 3) Preparation of biotin - labeled anti - AFP antibody:

An anti - AFP antibody (produced by KYOTO DAI -ICHI KAGAKU Co.) was dissolved in 0.1 mol/liter sodium hydrogencarbonate (hereinafter referred to as NaHCO3) to prepare its solution having a concentration of 1 mg/liter. NHS-LC-biotin was dissolved in dimethylsulfoxide to prepare its solution having a concentration of 3 g/liter. 0.1 ml of the solution of NHS - LC - biotin was added to 0.9 ml of the solution of anti-AFP antibody and reacted for 2 hours at room temperature. The reaction mixture was put into a dialyzing device equipped with a cellophane membrane and dialyzed therein for a total of three times against a solution of 0.01 mol/liter NaPB (pH 8.0) of 100 times as large as the mixture. The resulting dialysate was thus adjusted to have a concentration of 0.01 mg/ml. Thus, a biotin-labeled anti - AFP antibody was obtained.

### 4) Preparation of washing liquid:

2.1 g of citric acid monohydrate (produced by NAKARAITESK Co.), 10 ml of 30 % aqueous hydrogen peroxide (produced by NAKARAITESK Co.) and 1 g of Tween 20 (produced by NAKARAITESK Co.) were dissolved in a suitable amount of pure water. The pH of the resulting solution was adjusted to 5.2 by adding 1 N-NaOH (produced by NAKARAITESK Co.) thereto, using a pH meter. Pure water was added thereto to make 1 liter.5) Preparation of AFP solution:

AFP was dissolved in a solution of 0.1 mol/liter NaPB (pH 8.0) to prepare AFP solutions having a concentration of 0 ng/ml, 0.1 ng/ml and 1 ng /ml.

### 6) Measurement of AFP:

6) -1 Step of forming immunocomplex:
   0.1 ml of the Acridinium I-labeled anti - AFP antibody, 0.1 ml of the biotin-labeled anti-AFP antibody and 0.8 ml of the AFP solution having the concentration varying as above were mixed and reacted for 30 minutes at room temperature.
6) -2 Step of fixing the immunocomplex to solid phase:
   0.06 ml of each reaction mixture prepared above was applied to the avidin - bonded glass fiber filter paper as cut to have a diameter of 9 mm and reacted for 10 minutes at room temperature.
6) -3 Step of washing the solid phase and converting the acridinium to luminescent one:
   A filter paper was put into a Buchener funnel, the previously-reacted, avidin-bonded glass fiber filter paper was put on the filter paper, and 10 ml of the washing liquid prepared above was poured over this to wash it therewith while sucking the washing liquid using a water jet pump. After thus washed, this was left at room temperature for 15 minutes.
6) -4 Step of starting light emission and measuring the amount of light emitted:
   0.04 ml of 1 N-NaOH was added to the filter paper processed above to make it emit light, whereupon the amount of the light emitted was measured using an ultra - minor light metering device (produced by KYOTO DAI-ICHI KAGAKU Co.).

The results obtained in Example 1 are shown in Table 1.

**TABLE 1**

| AFP Concentration (ng/ml) | | | |
|---|---|---|---|
| | 0 | 0.1 | 1 |
| 1 | 1955 | 4996 | 26162 |
| 2 | 2279 | 4570 | 25690 |
| 3 | 2156 | 5491 | 24495 |
| 4 | 1981 | 4535 | 25050 |
| 5 | 2136 | 4709 | 25961 |
| 6 | 1790 | 4864 | 27376 |
| 7 | 2406 | 4688 | 24741 |
| 8 | 2384 | 5307 | 27361 |
| 9 | 2120 | 4921 | 26158 |
| 10 | 2194 | 5036 | 22387 |
| Mean Value | 2140 | 4912 | 25528 |
| SD | 193 | 310 | 1475 |
| CV % | 9.02 | 6.32 | 5.77 |
| (unit : number of counts) | | | |

In the table, SD and CV indicate the standard deviation and the coefficient of variation, respectively. The same shall apply to the tables mentioned hereinunder.

### Example 2:

The same process as in Example 1 was repeated, except for the following changes.5) Preparation of AFP solution:

As the diluent liquid, the washing liquid (pH 5. 2) as prepared in Example 1 was used in place of NaPB (pH 8.0).6) -3 Step of washing the solid phase and converting the acridinium to luminescent one:

In place of the corresponding step in Example 1 where the washed system was left as it was for a while before the next step, the next step was " immediately" started for making the system emit light and measuring the amount of the light emitted

The results obtained in Example 2 are shown in Table 2.

**TABLE 2**

| AFP Concentration (ng/ml) | | | |
|---|---|---|---|
| | 0 | 0.1 | 1 |
| 1 | 2349 | 10445 | 69409 |
| 2 | 2258 | 11123 | 64789 |
| 3 | 1996 | 11896 | 65284 |
| 4 | 2349 | 11370 | 64644 |
| 5 | 2069 | 12658 | 66517 |
| 6 | 2273 | 9924 | 67480 |
| 7 | 2203 | 10072 | 58513 |
| 8 | 2040 | 10661 | 66957 |
| 9 | 2074 | 10014 | 54732 |
| 10 | 2189 | 10712 | 64792 |
| Mean Value | 2180 | 10888 | 64312 |
| SD | 128 | 887 | 4407 |
| CV % | 5.87 | 8.14 | 6.85 |
| (unit: number of counts) | | | |

### Example 3:

Thyroid stimulating hormone (hereinafter referred to as TSH) was measured in accordance with the following method.

### 1) Preparation of avidin - bonded glass fiber filter paper:

The avidin - bonded glass fiber filter paper as prepared in Example 1 was used.

### 2) Preparation of Acridinium I-labeled anti-TSH antibody:

An Acridinium I-labeled anti-TSH antibody was prepared in the same manner as in Example 1, except that the anti - AFP antibody was replaced by an anti - TSH antibody (produced by Medics Biochemica Co.).

### 3) Preparation of biotin - labeled anti - TSH antibody:

A biotin - labeled anti - TSH antibody was prepared in the same manner as in Example 1, except that the anti-AFP antibody was replaced by an anti-TSH antibody (produced by Medics Biochemica Co.).

### 4) Preparation of washing liquid:

The washing liquid as prepared in Example 1 was used.

### 5) Preparation of TSH solution:

TSH was dissolved in the washing liquid to prepare TSH solutions having a concentration of 0 ng/ml, 0.01 ng/ml and 0.1 ng/ml.

### 6) Measurement of TSH:

6) -1 Step of forming immunocomplex:
   0.1 ml of the Acridinium I-labeled anti - TSH antibody, 0.1 ml of the biotin - labeled anti-TSH antibody and 0.8 ml of the TSH solution having the concentration varying as above were mixed and reacted for 30 minutes at room temperature.
6) -2 Step of fixing the immunocomplex to solid phase:
   0.06 ml of each reaction mixture prepared above was applied to the avidin - bonded glass fiber filter paper as cut to have a diameter of 9 mm and reacted for 10 minutes at room temperature.
6) -3 Step of washing the solid phase and converting the acridinium to luminescent one:
   A filter paper was put into a Buchener funnel, the previously - reacted, avidin - bonded glass fiber filter paper was put on the filter paper, and 10 ml of the washing liquid prepared above was poured over this to wash it therewith while sucking the washing liquid using a water jet pump.
6) -4 Step of starting light emission and measuring the amount of light emitted:
   Immediately after the previous step, 0.04 ml of 1 N-NaOH was added to the filter paper processed above to make it emit light, whereupon the amount of the light emitted was measured using an ultra - minor light metering device (produced by KYOTO DAI-ICHI KAGAKU Co.).

The results obtained in Example 3 are shown in Table 3.

**TABLE 3**

| TSH Concentration (ng/ml) | | | |
|---|---|---|---|
| | 0 | 0.01 | 0.1 |
| 1 | 2741 | 3860 | 15870 |
| 2 | 2863 | 4035 | 16855 |
| 3 | 2596 | 4054 | 17473 |
| 4 | 2205 | 3710 | 17301 |
| 5 | 2560 | 4290 | 16015 |
| 6 | 2550 | 4204 | 16644 |
| 7 | 2604 | 3797 | 16640 |
| 8 | 2909 | 4396 | 16346 |
| 9 | 2714 | 4252 | 17243 |
| 10 | 2522 | 3740 | 17853 |
| Mean Value | 2626 | 4034 | 16824 |
| SD | 199 | 247 | 645 |
| CV % | 7.58 | 6.14 | 3.83 |
| (unit: number of counts) | | | |

### Comparative Example 1:

The same process as in Example 1 was repeated, except for the following changes.

### 4) Preparation of washing liquid:

In place of the washing liquid (pH 5.2) prepared and used in Example 1, the following washing liquid was prepared and used.

Disodium hydrogenphosphate 12 - hydrate (produced by NAKARAITESK Co.) and 1 g of Tween 20 (produced by NAKARAITESK Co.) were dissolved in a suitable amount of pure water. The pH of the resulting solution was adjusted to 7.4 by adding 1 N-NaOH (produced by NAKARAITESK Co.) thereto, using a pH meter. pure water was added to this to make 1 liter.

### 6) -3 Step of washing the solid phase and converting the acridinium to luminescent one:

This step is different from the corresponding step in Example 1 where the washed system was left at room temperature for 15 minutes, in that the following acidifying buffer solution was added to the system that had been washed with the above -mentioned washing liquid in the pervious step and thereafter the system was left at room temperature for 15 minutes.

Citric acid monohydrate (produced by NAKARAITESK Co.) and 10 ml of 30 % aqueous hydrogen peroxide (produced by NAKARAITESK Co.) were dissolved in a suitable amount of pure water. The pH of the resulting solution was adjusted to 4.0 by adding 1 N - NaOH (produced by NAKARAITESK Co.) thereto, using a pH meter. Pure water was added to this to make 1 liter.

The results obtained in Comparative Example 1 are shown in Table 4.

**TABLE 4**

| AFP Concentration (ng/ml) | | | |
|---|---|---|---|
| | 0 | 0.1 | 1 |
| 1 | 1430 | 4411 | 21380 |
| 2 | 1788 | 5260 | 20630 |
| 3 | 1566 | 4927 | 21476 |
| 4 | 1413 | 3824 | 19361 |
| 5 | 1925 | 4924 | 20228 |
| 6 | 1461 | 5806 | 20810 |
| 7 | 1569 | 4706 | 21201 |
| 8 | 1466 | 5477 | 22771 |
| 9 | 1382 | 5353 | 21442 |
| 10 | 1768 | 5318 | 20688 |
| Mean Value | 1577 | 5001 | 20999 |
| SD | 187 | 577 | 900 |
| CV % | 11.9 | 11.5 | 4.29 |
| (unit: number of counts) | | | |

### Comparative Example 2:

The same process as in Example 3 was repeated, except for the following changes.

### 4) Preparation of washing liquid:

In place of the washing liquid (pH 5.2) in Example 3, the washing liquid (pH 7.4) as prepared in Comparative Example 1 was used.

### 6) -3 Step of washing the solid phase and converting the acridinium to luminescent one:

In place of the corresponding step in Example 3 where the washing step was "immediately" followed by the next step of starting light emission and measuring the amount of light emitted, the same step as in Comparative Example 1 was employed.

The results obtained in Comparative Example 2 are shown in Table 5.

**TABLE 5**

| TSH Concentration (ng/ml) | | | |
|---|---|---|---|
| | 0 | 0.01 | 0.1 |
| 1 | 1535 | 3172 | 13283 |
| 2 | 1839 | 3533 | 13142 |
| 3 | 1930 | 3294 | 10695 |
| 4 | 1977 | 2960 | 12221 |
| 5 | 1892 | 3257 | 13070 |
| 6 | 2052 | 3076 | 13509 |
| 7 | 1600 | 2957 | 10708 |
| 8 | 1782 | 2889 | 13401 |
| 9 | 2158 | 2332 | 13202 |
| 10 | 2121 | 3206 | 12468 |
| Mean Value | 1889 | 3068 | 12570 |
| SD | 207 | 322 | 1063 |
| CV % | 11.0 | 10.5 | 8.46 |
| (unit: number of counts) | | | |

Evaluation of the results obtained in Examples and Comparative Examples:

Example 1: The dispersion in the low-concentration data measured in Example 1 is much smaller than that in Comparative Example 1.

Example 2: The dispersion in the data measured in Example 2 is comparable to that in Example 1. However, the absolute data of light emission measured in Example 2 are larger than those in Example 1. In addition, Example 2 verified that, when the diluent liquid used has pH falling between 5 and 6, the rest time between the washing step and the next step for light emission is unnecessary.

Example 3: The data of light emission measured in Example 3 are larger than those in Comparative Example 2. In addition, the dispersion in the data measured in Example 3 is much smaller than that in Comparative Example 2.

## Claims

1. An immunological chemiluminescence method using an acridinium ester as the labeling substance for quantitatively determining the substance to be measured to be contained in a sample, which comprises the following steps in that order:
(a) a step of diluting the sample with a diluent liquid to a desired concentration,
(b) a step of bonding a reagent containing an acridinium ester-labeled antibody (or antigen) capable of specifically recognizing the substance to be measured to be in the sample and a physiologically-active substance that has been treated so as to be able to be fixed on a solid phase, to the substance to be measured to be in the sample by antigen-antibody reaction to thereby form an immunocomplex,
(c) a step of fixing the immunocomplex on a solid phase,
(d) a step of separating the antibody (or antigen) that has not participated in the reaction from the reacted, acridinium ester-labeled antibody (or antigen), using a washing liquid, while converting the acridinium ester of a non-luminescent type into that of a luminescent type, and
(e) a step of adding an alkaline solution to the reaction system to thereby make the system emit light, followed by measuring the amount of the light thus emitted, the method being characterized in that, of the diluent liquid to be used in the step (a), the reagent to be used in the step (b) and the washing liquid to be used in the step (d), at least the washing liquid has pH of from 5 to 6.

2. The chemiluminescence method as claimed in claim 1, wherein the substance to be measured is an antigen.

3. The chemiluminescence method as claimed in claim 1, wherein the substance to be measured is an antibody.

4. The chemiluminescence method as claimed in claim 1, wherein, of the diluent liquid to be used in the step (a), the reagent to be used in the step (b) and the washing liquid to be used in the step (d), the reagent and the washing liquid have pH of from 5 to 6.

5. The chemiluminescence method as claimed in claim 1, wherein, of the diluent liquid to be used in the step (a), the reagent to be used in the step (b) and the washing liquid to be used in the step (d), the diluent liquid and the washing liquid have pH of from 5 to 6.

6. The chemiluminescence method as claimed in claim 1, wherein the diluent liquid to be used in the step (a), the reagent to be used in the step (b) and the washing liquid to be used in the step (d) each have pH of from 5 to 6.

7. The chemiluminescence method as claimed in claim 1, 2, 3,4,5 or 6, wherein the step (a) is omitted if the dilution of the sample is unnecessary.
